(19) Europäisches Patentamt
European Patent Office
Office européen des brevets

(11) **EP 2 853 160 A1**

(12) **EUROPEAN PATENT APPLICATION**

(43) Date of publication:
**01.04.2015 Bulletin 2015/14**

(21) Application number: **14186253.2**

(22) Date of filing: **24.09.2014**

(51) Int Cl.:
*A01N 65/08* [(2009.01)]     *A61K 36/82* [(2006.01)]
*B01D 46/00* [(2006.01)]     *A61K 36/258* [(2006.01)]
*A01N 25/34* [(2006.01)]     *A01P 1/00* [(2006.01)]

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.09.2013   KR 20130114045**

(71) Applicants:
• **LG ELECTRONICS INC.**
**Yeongdeungpo-Gu**
**Seoul 150-721 (KR)**

• **Pukyong National University Industry-University Cooperation**
**Busan 607-739 (KR)**

(72) Inventors:
• **Lee, Sunghwa**
**153-802 Seoul (KR)**
• **Lee, Myungsuk**
**603-737 Busan (KR)**

(74) Representative: **Vossius & Partner**
**Siebertstrasse 3**
**81675 München (DE)**

(54) **Antimicrobial composition comprising ginseng extract and green tea extract and an antimicrobial filter containing it**

(57)     Discloses an synergistic antimicrobial composition containing a ginseng extract and a green tea extract in a weight ratio from 6:4 to 7:3 and an antimicrobial filter containing said composition and a method for manufacturing them. Said single filter exhibits higher antimicrobial and antiviral efficiency, has little pressure loss and could be produced at lower cost as compared with two systems containing two filters with single antimicrobial agent.

EP 2 853 160 A1

**Description**

## BACKGROUND OF THE INVENTION

### Field of the Invention

The present invention relates to an antimicrobial composition containing a ginseng extract and a green tea extract, an antimicrobial filter and a method for manufacturing the same.

### Discussion of the Related Art

In response to recently increased interest in the environment and thus increased demand for indoor air cleaning, a variety of air conditioning devices for removing foreign matter and microorganisms from the air are developed.

Filters are used for such air conditioning devices. These filters require various forms and properties according to types, sizes and characteristics of objects to be removed. Accordingly, various filters suitable for these requirements have been developed.

When antimicrobial and antibacterial activities are imparted to such filters, the filters can sufficiently remove or sterilize microorganisms such as bacteria, viruses and fungi in the air, thus obtaining satisfactory air conditioning effects and excellent hygienic effects. The antimicrobial and antibacterial activities are further important in environments in which recirculation of air is required due to sealing for a long time and microorganisms are readily propagated.

Ginseng (*Panax ginseng C.A. Meyer*) which is a herbaceous perennial plant belonging to the family Araliaceae and genus Panax has been used as traditional medicines in the Asia, in particular, Korea, China and Japan, for many centuries. Ginseng is known to contain, as active ingredients, ginsenosides, polyacetylenes, alkaloids, phenolic compounds, polysaccharides, flavonoids, peptides, fatty acids and the like and have an antimicrobial function.

Green tea (*Camellia teaceac*) is known to provide nutrients such as vitamins and minerals and satisfy preference such as taste and flavors. A main ingredient imparting an astringent taste to green tea is catechin which belongs to polyphenols. Catechin is classified into epicatechin, epigallocatechin, epicatechin gallate, epigallocatechin gallate and the like according to derivatives thereof. Catethin is present in an amount of about 8 to 20% in dried green tea, is water-soluble and is released into water when dried green tea is boiled in water. Meanwhile, the green tea contains a variety of physiologically active substances. In particular, catechin is found to function to reduce LDL cholesterol in blood to suppress thrombosis and prevent arteriosclerosis, suppress formation of peroxide lipids and prevent fat oxidation and is also used as a natural antioxidant. In addition, green tea provides anti-aging functions, suppresses generation and propagation of cancers and prevents food poisoning due to antitoxic activity of inactivating toxins.

Disadvantageously, antimicrobial filters effective for bacteria and antiviral filters effective for viruses are separately used because antimicrobial filters using plant extracts developed to date are coated with a single substance. Antimicrobial filters developed to date have only a single function and thus have a problem of increased manufacturing costs or increased pressure loss when applied to products.

## SUMMARY OF THE INVENTION

Accordingly, the present invention is directed to an antimicrobial composition containing a ginseng extract and a green tea extract, an antimicrobial filter and a method for manufacturing the same that substantially obviate one or more problems due to limitations and disadvantages of the related art.

One object of the present invention is to provide an antimicrobial composition containing a ginseng extract and a green tea extract with highly efficient antibacterial and antiviral effects, an integral antimicrobial filter having excellent antibacterial and antiviral effects and little pressure loss and methods for manufacturing the same. In particular, another object of the present invention is to provide an antimicrobial composition containing a ginseng extract and a green tea extract at an optimum ratio to provide maximum antibacterial and antiviral effects, an antimicrobial filter and a method for manufacturing the same.

Additional advantages, objects, and features of the invention will be set forth in part in the description which follows and in part will become apparent to those having ordinary skill in the art upon examination of the following or may be learned from practice of the invention. The objectives and other advantages of the invention may be realized and attained by the structure particularly pointed out in the written description and claims hereof as well as the appended drawings.

To achieve these objects and other advantages and in accordance with the purpose of the invention, as embodied and broadly described herein, an antimicrobial composition contains a ginseng extract and a green tea extract as active ingredients.

The ginseng extract and the green tea extract may be present in a weight ratio of 60:40 to 70:30.

The ginseng extract may be selected from the group consisting of a ginseng leaf extract, a ginseng rootlet extract, a ginseng main root extract and a combination thereof.

The ginseng extract may be selected from the group consisting of a ginseng extract, a concentrated ginseng extract, a dried ginseng extract and a combination thereof and the green tea extract may be selected from the group consisting of a green tea extract, a concentrated green tea extract, a dried green tea extract and a combination thereof.

In another aspect of the present invention, a method for preparing the antimicrobial composition includes preparing a ginseng extract, preparing a green tea extract, and mixing the ginseng extract with the green tea extract.

The ginseng extract and the green tea extract may be mixed at a weight ratio of 60:40 to 70:30.

Each of the preparation of the ginseng extract and the preparation of the green tea extract may further include powderizing the prepared extract and the powderizing may be carried out by concentrating each of the extracts under reduced pressure.

The preparation of the ginseng extract may include grinding any one selected from the group consisting of ginseng leaves, ginseng rootlets, ginseng main roots and a combination thereof to prepare ground ginseng, mixing the ground ginseng with distilled water and sterilizing the resulting mixture, and drying the mixture and extracting the dried mixture with a solvent.

The preparation of the green tea extract may include grinding green tea to prepare ground green tea, mixing the ground green tea with distilled water and sterilizing the resulting mixture, and drying the mixture and extracting the dried mixture with a solvent.

In another aspect of the present invention, an antimicrobial filter contains a ginseng extract and a green tea extract.

The ginseng extract and the green tea extract may be present in a weight ratio of 60:40 to 70:30.

The antimicrobial filter may further include any one selected from the group consisting of an antimicrobial substance, a binder, a metal and a combination thereof.

The antimicrobial substance may include any one selected from the group consisting of glutaraldehyde, phthalaldehyde (PA), potassium disulfite (PD), bronopol, sodium deoxycholate, quaternary ammonium and a combination thereof.

The binder may include any one selected from the group consisting of polyvinylpyrrolidone iodine (PVPI), norspermidine (NS, bis(3-aminopropyl)octylamine) and a combination thereof.

The binder may include any one selected from the group consisting of a silicone-modified epoxy resin, a silicone-modified acrylic resin, a silicone-modified urethane resin, a silicone-modified vinyl resin, a polyvinyl alcohol silicone resin, a urethane resin, an acrylic resin, a vinyl resin, a silicone resin and a combination thereof.

The metal may include any one selected from the group consisting of Ag, Cu, Zn, Ca, Mn and a combination thereof.

The antimicrobial filter may be used for an air conditioner, an air cleaner, a refrigerator or a facial treatment device.

It is to be understood that both the foregoing general description and the following detailed description of the present invention are exemplary and explanatory and are intended to provide further explanation of the invention as claimed.


**DETAILED DESCRIPTION OF THE INVENTION**

Hereinafter, the present invention will be described in detail with reference to embodiments such that the present invention can be easily implemented by those having an ordinary skill in the art. However, the present invention may be implemented in various different forms and is not limited to embodiments described herein.

Unless otherwise mentioned, the term "antimicrobial" means removal of bacteria as well as removal of fungi and viruses.

The antimicrobial composition according to an embodiment of the present invention contains a ginseng extract and a green tea extract wherein the ginseng extract and the green tea extract is preferably present in a weight ratio of 60:40 to 70:30.

The ginseng extract means an extract of ginseng and includes an extract of unprocessed ginseng as well as processed ginseng such as red ginseng or black ginseng.

The ginseng extract may be selected from a ginseng rootlet extract extracted from ginseng rootlets, a ginseng leaf extract extracted from ginseng leaves, a ginseng main root extract extracted from ginseng main roots, a fermented ginseng rootlet extract obtained by fermenting and extracting ginseng rootlets, a fermented ginseng leaf extract obtained by fermenting and extracting ginseng leaves, and a fermented ginseng main root extract obtained by fermenting and extracting ginseng main roots and a combination thereof.

The ginseng extract is safe to humans and is effective in removing or sterilizing microorganisms. Specifically, the ginseng extract is highly effective in removing general bacteria as well as pathogenic bacteria such as food poisoning bacteria and is effective in removing viruses which are causes of infectious diseases such as avian influenza or swine flu, or fungi such as filamentous fungi which are difficult to remove due to propagation by spore, thus exhibiting excellent antimicrobial and antibacterial effects upon a wide range of microorganisms.

Furthermore, the ginseng extract is extracted from ginseng used as a medicine or food additive and is thus harmless to humans, thus being applicable to areas where humans and food come into direct contact.

When the ginseng extract is prepared using a ginseng by-product selected from the group consisting of ginseng rootlets, ginseng leaves and a combination thereof, the ginseng extract is prepared using ginseng by-products having low applicability as ginseng, the medicinal ingredient, or ginseng wastes, thereby advantageously providing improved economic efficiency and excellent environmental effects.

In particular, among the ginseng extracts, the fermented ginseng extract obtained by fermenting and extracting a ground ginseng product selected from the group consisting ginseng rootlets, ginseng main roots, ginseng leaves and a combination thereof can improve antimicrobial and antibacterial effects of ginseng by fermentation.

Furthermore, among the ginseng extracts, the ginseng leaf extract, the fermented ginseng rootlet extract and the fermented ginseng main root extract have considerably better antimicrobial and antibacterial effects than other extracts, and in particular, the ginseng leaf extract and the fermented ginseng rootlet extract have superior antimicrobial effects and excellent economical and environmental effects.

The ginseng extract may contain one selected from the group consisting of a ginseng extract, a concentrated ginseng extract, a dried ginseng extract and a combination thereof. When the concentrated ginseng extract or the dried ginseng extract is used as the ginseng extract, the ginseng extract can be easily stored and transported and can maintain antibacterial and antimicrobial functions. The dried ginseng extract may be a dried or freeze-dried powder of the ginseng extract.

The green tea extract means an extract of green tea and includes an extract of unprocessed green tea as well as an extract of processed green tea.

The green tea extract may be selected from a green tea extract extracted from green tea, a fermented green tea extract obtained by fermenting and extracting green tea and a combination thereof. The fermented green tea extract can improve antimicrobial and antibacterial effects of the green tea extract by fermentation.

The green tea extract may contain one selected from the group consisting of a green tea extract, a concentrated green tea extract, a dried green tea extract and a combination thereof. When the concentrated green tea extract or the dried green tea extract is used as the green tea extract, the green tea extract can be easily stored and transported and can maintain antibacterial and antimicrobial functions. The dried green tea extract may be a dried or freeze-dried powder of the green tea extract.

In the antimicrobial composition of the present invention, a weight ratio of the ginseng extract to the green tea extract is 50:50 to 80:20, preferably 60:40 to 70:30, even more preferably 62:38 to 68:32, most preferably 66:34. When the ginseng extract and the green tea extract are mixed at the ratio, maximum antibacterial and antiviral effects can be obtained. When the weight ratio of ginseng extracts is lower than the range defined above, for example, antibacterial activity against *E. coli* is remarkably reduced and when the weight ratio of the green tea extracts is less than the range defined above, for example, a function deterioration problem in which feline calcivirus (FCV) cannot be removed within a short time, for example, 3 hours, preferably 2 hours occurs.

The antimicrobial composition according to the present invention further comprises at least one additive in addition to the active ingredients and the additives can be freely selected without limitation according to composition of stock solution.

The amount of active ingredients containing the ginseng extract and the green tea extract is 0.01 to 100% by weight, preferably 0.1 to 70% by weight, more preferably 1 to 10% by weight, with respect to the total weight of the composition, but the present invention is not limited thereto. Water or the like as well as the active ingredients and the additive is added to adjust a total weight to 100% by weight so that a stock solution having a desired composition can be prepared.

In addition, a variety of formulations are prepared using the active ingredient or the stock solution containing the active ingredient. The formulations may be any type of formulations prepared in the art and examples thereof include, but are not limited to, solutions, emulsions, suspensions, pastes, gels, creams, lotions, soaps, oils, powders, waxes, sprays and the like.

The method for preparing the antimicrobial composition according to an embodiment of the present invention includes: preparing a ginseng extract; preparing a green tea extract; and mixing the ginseng extract with the green tea extract, wherein the mixing is preferably carried out by mixing the ginseng extract with the green tea extract at a weight ratio of 60:40 to 70:30. The preparation of the ginseng extract and the preparation of the green tea extract may further include powderizing the prepared ginseng and green tea extracts, respectively, and the powderization is for example carried out by concentrating the resulting extracts under reduced pressure. The preparation of the ginseng extract and the preparation of the green tea extract may be exchanged in order or simultaneously performed.

The preparation of the ginseng extract may include grinding any one selected from the group consisting of ginseng leaves, ginseng rootlets, ginseng main roots and a combination thereof to prepare a ground ginseng (grinding);

mixing the ground ginseng with distilled water and sterilizing the resulting mixture (sterilization); air-cooling the sterilized mixture; and drying the mixture and extracting the dried mixture with a solvent (extraction) and the preparation of the green tea extract may include grinding green tea to prepare a ground green tea (grinding); mixing the ground green tea with distilled water and sterilizing the resulting mixture (sterilization); air-cooling the sterilized mixture; and drying the mixture and extracting the dried mixture with a solvent (extraction).

Any device for the grinding may be used so long as it is capable of grinding the ginseng leaves, ginseng rootlets, ginseng main roots or green tea and the device is for example a homogenizer. The size of ground ginseng or green tea product is not particularly limited and may be applied without limitation so long as it improves efficiency of sterilization and extraction.

Any method for sterilization may be used so long as it is capable of sterilizing the ground ginseng or green tea product and may be high-pressure steam sterilization in which the mixture prepared by mixing the ground ginseng or green tea product with distilled water is sterilized under high-pressure steam. The high-pressure steam sterilization is for example carried out by treating the mixture at 1.2 atm and at 121°C for 15 minutes or longer. The sterilization removes unnecessary microorganisms contained in the ground ginseng or green tea product and enables easy extraction from the ground ginseng or green tea product.

The air-cooling is for example cooling the sterilized mixture and is for example carried out by standing the mixture at room temperature for 12 hours.

In the extraction, the ground ginseng or green tea product is mixed with a solvent and is then extracted. Any solvent may be used without particular limitation so long as it is capable of dissolving the ground ginseng and green tea product and the solvent is specifically water, methanol, ethanol or the like and is preferably methanol. A process for preparation of the ginseng extract or green tea extract may be used without particular limitation so long as active ingredients can be extracted from the ground ginseng and green tea products and is for example extraction under stirring in a mantle while mixing with methanol. The extraction is carried out at a temperature of 40 to 100°C, preferably 50 to 75°C. The extraction may be repeated two, three or four times.

The preparation method may further include powderizing the prepared ginseng and tea extract extracts after extraction, and the powderization may include concentrating and powderizing the extracted ginseng or green tea extract to prepare a ginseng or green tea extract powder and is specifically carried out by concentrating and powderizing the ginseng extract or the green tea extract by concentration under reduced pressure.

The antimicrobial filter according to another embodiment of the present invention contains a ginseng extract and a green tea extract and a weight ratio of the ginseng extract to the green tea extract is preferably 60:40 to 70:30. In addition, the antimicrobial filter may further contain any one selected from the group consisting of an antimicrobial substance, a binder, a metal and a combination thereof.

Any antimicrobial substance may be used without limitation so long as it is known to have sterilization, antimicrobial or antibacterial effects.

Preferably, the antimicrobial substance may include any one selected from the group consisting of glutaraldehyde, phthalaldehyde (PA), potassium disulfite (PD), bronopol, sodium deoxycholate, quaternary ammonium and a combination thereof. These substances used as antimicrobial substances are demonstrated to be safe enough to be used in foods and improve antimicrobial and antibacterial effects of the filter and are harmless to humans. In particular, glutaraldehyde is effective in inhibiting activity of microorganisms by linking proteins and thereby immobilizing enzymes, and the sodium deoxycholate is effective in inhibiting cell growth by affecting cell membranes, and the quaternary ammonium increases pH and thereby has negative effects on survival conditions of microorganisms.

The binder fixes the ginseng extract or the green tea extract on carriers supporting the ginseng or green tea extract or filters having the function of the carriers as well as the effect of removing foreign matter through general air filtering and binds the ginseng extract or green tea extract on the carriers or filters in spite of air pressure loads of carriers or filters.

Any binder may be used without particular limitation so long as it is capable of binding the ginseng extract or green tea extract on the carriers or filters.

The binder may be an antimicrobial binder having both adhesion force and antimicrobial function, such as polyvinylpyrrolidone iodine (PVPI), norspermidine (NS, bis(3-aminopropyl)octylamine) and a combination thereof.

The binder can be an organic-inorganic hybrid binder such as silicon-modified epoxy resin, silicon-modified acrylic resin, silicon-modified urethane resin, silicon-modified vinyl resin, polyvinyl alcohol silicone resin, urethane resin, acrylic resin, vinyl resin, silicone resin or a combination thereof, preferably silicon-modified epoxy resin.

In the organic-inorganic hybrid binder, a silane compound such as organosilane or alkoxysilane for silicon modification of the binder, is selected from the group consisting of methyltrichlorosilane, vinyltrichlorosilane, 3-glycidoxypropyltrimethoxysilane, tetraethyl orthosilicate, 3-aminopropyltrimethoxysilane, 3-methacryloxypropyltri-methoxysilane, vinyltrimethoxysilane and combinations thereof.

The silicon-modified organic-inorganic hybrid binder may be prepared by hydrolyzing the silane compound with the binder resin in the presence of a solvent such as water or alcohol. In this process, a hydrolysis catalyst may be used.

The hydrolysis catalyst may be an inorganic acid such as hydrochloric acid, sulfuric acid or nitric acid.

The binder may be used as a combination of the antimicrobial binder and the organic-inorganic hybrid binder. In this case, the binder has antimicrobial or antibacterial activities while sufficiently fixing the ginseng extract or green tea extract on the carriers or filters, thus improving antimicrobial or antibacterial functions of the filter.

In particular, when polyvinylpyrrolidone iodine (PVPI) is used as the binder, sufficient adhesive effect can be obtained in spite of using a small amount of the binder and, at the same time, the improvement of antimicrobial or antibacterial property may be achieved as well due to the superior antibacterial property.

In addition, the antimicrobial filter may further contain a carrier and any carrier may be used without limitation so long as it is capable of supporting or coating the ginseng extract or the green tea extract to fix the same. There is no limitation as to type, shape, size and production method of the carrier so long as the carrier is contained in the filter or has the inherent function of the filter.

The carrier may be composed of fabrics, metals, plastics and the like and may include any one selected from the group consisting of non-woven fabrics, paper fibers, glass fibers, ion exchange fibers, cellulose fibers, asbestos fibers, activated carbon, titanium dioxide, zinc, copper, aluminum and combinations thereof.

The carrier may be produced as a variety of shapes including honeycomb, particle, net, filter paper, cotton, mesh, plate and groove shapes and these shapes may be modified or combined with one another.

In addition, the carrier may be a deodorization filter such as an activated carbon filter used in conventional home appliances such as refrigerators and air conditioners, a HEPA filter or a filter for air conditioners of vehicles, or may contain the same.

The non-woven fabric may be a dry or wet non-woven fabric and the dry non-woven fabric may include polypropylene (PP), polyethylene terephthalate (PET), polyethylene (PE), polyamide, nylon and a combination thereof.

In addition, the carrier may be an air filter. Any air filter may be used so long as it is used as a conventional air filter. Specifically, the carrier may be a non-woven fabric including polypropylene and may be a zebra-shaped filter.

In particular, when the air filter serving as the carrier and having the effect of removing foreign matter such as dust by general air filtering is used as the antimicrobial filter of the present invention, it imparts both air conditioning function and antimicrobial and antibacterial properties to the air conditioning filter, thus improving performance of air conditioning filter.

The antimicrobial filter may further include a metal. In this case, antimicrobial and antibacterial performance of the air conditioning filter can be improved. The metal may include any one selected from the group consisting of Ag, Cu, Zn, Ca, Mn and a combination thereof and may be a metal sol form.

The antimicrobial filter may have a configuration in which the ginseng extract, the green tea extract, the antimicrobial substance, the binder and the metal are supported or coated on the carrier. The supporting or coating may be carried out by a well-known method, for example, dipping, rolling, spraying and the like, but the present invention is not limited thereto. When the ingredients are coated on the carrier, the coating layer may have a thickness of several nanometers to several micrometers.

The antimicrobial filter may contain the ginseng extract and the green tea extract in an amount of 0.001 to 30% by weight, preferably 0.1 to 10% by weight, more preferably 0.3 to 5% by weight, based on the total weight of the ingredients. When the content of the ginseng extract and the green tea extract is less than 0.001% by weight, the antimicrobial activity may be insufficient and when the content exceeds 30% by weight, economic efficiency may be deteriorated. In addition, when the antimicrobial filter contains the ginseng extract and the green tea extract in the content range defined above, harmlessness to humans and sufficient antimicrobial activity can be imparted to the air conditioning filter coated with the same.

The antimicrobial substance is present in an amount of 0.0001 to 10% by weight, preferably 0.01 to 5% by weight, more preferably 0.08 to 3% by weight, with respect to the total weight of the ingredients. When the antimicrobial substance is used in an amount less than 0.0001% by weight, reinforced antimicrobial activity is disadvantageously deteriorated and when used in an amount higher than 10% by weight, improvement in antimicrobial effect as compared to the used amount is insufficient. Accordingly, the range defined above is preferably maintained.

The binder may be present in an amount of 0.001 to 40% by weight, preferably 0.01 to 30% by weight, based on the total weight of the ingredients. When the binder is used in an amount less than 0.001% by weight, it is disadvantageously difficult to fix the extract on the carrier or the filter, and when the binder is used in an amount higher than 40% by weight, it may have a negative effect on viscosity. The range defined above is preferably maintained.

The antimicrobial filter is for example obtained by applying, to a carrier, a coating solution containing 1 to 10% by volume of ginseng extract and green tea extract solutions prepared by dissolving the ginseng extract and the green tea extract in a first solvent selected from the group consisting of water, ethanol and a combination thereof, 0.05 to 1% by volume of a antimicrobial substance, 1 to 10% by volume of a metal, and 79 to 93% by volume of a binder solvent consisting of a binder and a second solvent. When the coating solution is applied, miscibility and coatability of the coating solution can be improved.

The binder solvent may contain the binder and the second solvent in amounts of 20 to 30% by volume and 70 to 80% by volume, respectively, and the second solvent may be any one selected from the group consisting of water (pure water), ethanol and a combination thereof.

The method may further include suitably drying the coating solution when the coating solution is supported or coated on the carrier.

The ingredients may be present in amounts enabling antimicrobial performance and adhesion performance (the capability of coating the ingredients on the carrier to maintain the antimicrobial performance). Specifically, a weight ratio of the total weight of the ginseng extract, the green tea extract and antimicrobial substance to the binder is 1:2 to 1:80, preferably, 1:5 to 1:40, most preferably 1:7.5 to 1:10. When the ingredients are used in the weight ratio defined above, antimicrobial performance and adhesive performance required for the air conditioning filter are optimized and economic efficiency is improved. The metal may be present in an amount of 1 to 10-fold by weight, based on the total weight of the ginseng extract and the green tea extract and the antimicrobial substance may be present in an amount of 0.01 to 10-fold by weight, based on the total weight of the ginseng extract and the green tea extract.

The antimicrobial filter contains the ginseng extract and the green tea extract, thus being harmless to humans, exhibiting superior antimicrobial and antibacterial effects and having air conditioning function as well as the effect of potently removing microorganisms due to effects of efficiently removing general bacteria as well as pathogenic bacteria, such as food poisoning bacteria, viruses and fungi, and thus deodorization effect.

Due to these properties, any antimicrobial filter may be applied to any device requiring the air conditioning filter, for example, home appliances such as air conditioners, air cleaners, a refrigerator, facial treatment devices and stylers, or vehicle air conditioners for circulating indoor air. In addition, the antimicrobial filter may be used as a filter such as a deodorization filter or a HEPA filter or may be used in conjunction therewith.

## **Example**

Hereinafter, the present invention will be described in detail with reference to embodiments such that the present invention can be easily implemented by those having an ordinary skill in the art. However, the present invention may be implemented in various different forms and is not limited to embodiments described herein.

### **<Preparation Example: Preparation of mixture of ginseng extract and green tea extract>**

#### (1) Preparation of active ingredient extract of ginseng

Ginseng rootlets and ginseng leaves were ground using a homogenizer to prepare a ground ginseng product (grinding). 30g of the ground ginseng was mixed with 600 ml of distilled water to prepare a mixture and the mixture was sterilized at 121°C for 15 minutes using a high-pressure steam sterilizer (sterilization). The sterilized mixture was cooled in the air. The air-cooled mixture was dried, and 600g of the ground powder was incorporated in 6L of methanol and stirred and extracted three times in a mantle for 3 hours, followed by filtering, to prepare a ginseng extract (extraction). The ginseng extract was concentrated under reduced pressure using a rotary vacuum evaporator and a 45°C constant-temperature bath and then powderized to prepare a ginseng extract powder (powderization).

#### (2) Preparation of active ingredient extract of green tea

Green tea was ground using a homogenizer to prepare a ground green tea product (grinding). 30g of the ground green tea product was mixed with 600 ml of distilled water to prepare a mixture and the mixture was sterilized at 121 °C for 15 minutes using a high-pressure steam sterilizer (sterilization). The sterilized mixture was cooled in the air. The air-cooled mixture was dried and 600g of the ground powder was incorporated in 6L of methanol and stirred and extracted three times in a mantle for 3 hours, followed by filtering, to prepare a ginseng extract (extraction). The green tea extract was concentrated under reduced pressure using a rotary vacuum evaporator and a 45°C constant-temperature bath and then powderized to prepare a green tea extract powder (powderization).

#### (3) Preparation of mixture of ginseng extract and green tea extract

The ginseng extract powder and the green tea extract powder obtained in (1) and (2) were dissolved in de-ionized water such that solutions having concentrations of 5 mg/ml were obtained. The ginseng extract and the green tea extract dissolved in distilled water were mixed at various ratios and were used in the following process.

**<Test Example: Method used for testing antimicrobial and antiviral performances>**

1. Evaluation of antimicrobial performance using shaking flask method

A shaking flask method was used to test antimicrobial effects against *E. coli* and *S. aureus.* A maintenance medium inoculated with about $10^5$ (cfu/ml) of *E. coli* or *S. aureus* was prepared using 1% nutrient broth and 0.5% NaCl. The substances of Comparative Example and Example were reacted with the maintenance medium at a temperature of 35°C. After a predetermined time, antimicrobial effect was evaluated using a pour plate method. The antimicrobial effect was calculated using the following equation:

$$Antimicrobial\ effect\ (\%) = (\frac{Mc - Me}{Mc})\ X\ 100$$

*Me: the number of bacteria in maintenance medium containing the substances*
*Mc: the number of bacteria in maintenance medium not containing the substances*

2. Evaluation of viral removal performance to feline calcivirus (FCV)

Antiviral performance was evaluated using a Feline calcivirus (FCV F9 strain, cat. # VR-782-ATCC) which is similar genetic structure and physical and chemical properties to norovirus and belongs to the same group, Caliciviridae.
Virus removal effect was identified by checking infection according to virus reaction time using Crandell's feline kidney (CrFK) cells (ATCC, CCL-94).
An Eagle's minimum essential medium MEM (10% FBS or HS) was added to a 75 $cm^2$ cell culture flask and cultured in a 5% $CO_2$ incubator at 37°C for one night. The cells were subcultured every two days, inoculated with FCV F9 strains and cultured in a 5% $CO_2$ incubator at 37°C, and whether or not the virus was cultured in the prepared sample was identified to prepare cells and viruses.
CrFK cells were seeded at a density of $1.3 \times 10^4/100$ ul on a 96 well cell culture plate and cultured in a 5% $CO_2$ incubator at 37°C for one night.
On the next day, the prepared virus was 10-fold diluted with a maintenance medium by a decimal method. A growth medium was removed on a 96 well-plate composed of a single membrane using an aspirator and each of eight wells was inoculated with 25 $\mu\ell$ of the diluted virus.
The cells were inoculated with the virus in a 5% $CO_2$ incubator at 37°C for 90 minutes and 100 $\mu\ell$ of a maintenance medium was added to each well. A viral titer was determined by comparing the amount of viruses left after reacting with the extract for a predetermined time with a control group and calculating TCID (tissue culture infectious dose)$_{50}$ measured in CrFK cells.
On the fifth day of viral culture, cells dissolved by viruses were visualized and a dilution process of wells exhibiting 50% or more of cytopathic effect (CPE) was determined by the Reed-Munch method and was represented as Log TCID$_{50}$.

**<Comparative Example 1: Use of green tea extract alone>**

Antimicrobial performance was evaluated using green tea extract alone by the method according to Test Example. Results are shown in Table 1 below.

TABLE 1

| Test results of antimicrobial performance of green tea extract | | | | | |
|---|---|---|---|---|---|
| Test strains and reaction time sample | | *E. coli* (24hr) | *S. aureus* | *A. niger* (48hr) | FCV (1hr) |
| Green tea extract | Non-fermentation | 0% | 99.98% (24hr) | 28.57% | 100% |
| | Fermentation | 0% | 100% (3hr) | 33.33% | 100% |

As can be seen from Table 1 above, the green tea extract was found to inactivate 100% FCV after reacting with FCV for one hour or have no antimicrobial activity against E. coli.

**<Comparative Example 2: Use of ginseng extract alone>**

Antimicrobial performance was evaluated using the ginseng extract alone by a method according to Test Example. Results are shown in Table 2 below.

TABLE 2

| Test results of antimicrobial performance of ginseng extract | | | | |
|---|---|---|---|---|
| Test strains and Reaction time Sample | *E. coli* (24hr) | *S. aureus* (3hr) | *A. niger* (48hr) | FCV (6hr) |
| Ginseng extract | 100% | 100% | 90.95% | 100% |

As can be seen from Tables 1 and 2 above, when the ginseng extract was used alone, 100% of *E. coli* and *S. aureus* were removed within 24 hours and 100% of FCV (tested instead of norovirus) was inactivated after reaction for 6 hours. That is, regarding antiviral activity, the ginseng extract exhibited about a 6-fold longer time required for 100% inactivation than the green tea extract.

Consequently, as can be seen from Tables 1 and 2, when the respective extracts were used alone, they did not exhibit complete and rapid antimicrobial and antibacterial effects against all strains.

**<Examples 1 to 5: Use of combination of ginseng extract and green tea extract>**

Antimicrobial performance of a mixture of the ginseng extract (ginseng) and the green tea extract (green tea) was tested. The results shown in Tables 3 and 4 below were obtained.

TABLE 3

| Test results of antimicrobial performance of combination of ginseng and green tea extracts on *E. coli* and *Staphylococcus Aureus* | | | | |
|---|---|---|---|---|
| Strain and reaction time ginseng:green tea (weight ratio) | E. coli (Escherichia coli) | | Staphylococcus Aureus | |
| | 5 hr | 24 hr | 3 hr | 5 hr |
| (Comparative Example 2) 100:0 | 99.96% | 100% | 100% | 100% |
| (Example 1) 90:10 | 99.87% | 99.99% | 100% | 100% |
| (Example 2) 80:20 | 99.71% | 99.99% | 100% | 100% |
| (Example 3) 66:34 | 99.24% | 99.99% | 100% | 100% |
| (Example 4) 50:50 | 98.99% | 99.99% | 100% | 100% |
| (Example 5) 34:66 | 98.68% | 99.99% | 100% | 100% |
| (Comparative Example 1) 0:100 | 0% | 0% | 100% | 100% |

TABLE 4

| Test results of antimicrobial performance of combination of ginseng and green tea extracts on FCV (TCID$_{50}$) | | | | | |
|---|---|---|---|---|---|
| Reaction time ginseng:green tea(weight ratio) | 30 min | 1 hr | 2 hr | 3 hr | 6 hr |
| (Comparative Example 2) 100:0 | - | 98.83% | 97.50% | 97.50% | 100% |
| (Example 1) 90:10 | - | 95.28% | 96.00% | 98.67% | 100% |
| (Example 2) 80:20 | - | 97.67% | 99.86% | 100% | 100% |
| (Example 3) 66:34 | - | 98.93% | 100% | 100% | 100% |
| (Comparative Example 1)0:100 | 98.00% | 100% | 100% | 100% | 100% |
| * "-" in Table 4 indicates that antimicrobial effect was not determined. | | | | | |

As can be seen from Table 3 above, upon reaction for 24 hours, 100% of E. coli was removed when the ginseng extract was used alone, and 99.99%, 99.99% and 99.97% of E. coli was removed when the ginseng extract and the green tea extract were used at a ratio of 90:10, 80:20 and 66:34, respectively. 100% of *Staphylococcus Aureus* was removed upon reaction for three hours under all the mixing ratio conditions.

In addition, as can be seen from Table 4, as a result of measurement of antiviral performance, 100% inactivation was observed upon reaction for one hour when the green tea extract was used alone, 100% inactivation was observed upon reaction for six hours when the ginseng extract was used alone, and 100% inactivation was observed upon reaction for six, three and two hours when the ginseng extract and the green tea extract were combined in ratios of 90:10, 80:20 and 66:34, respectively.

The results indicated that, when the ginseng extract and the green tea extract were mixed at a weight ratio of 66:34 (Example 3), 99.99% (24 hr) and 100% (3 hr) of *E. coli* and *S. aureus* were removed, respectively and 100% inactivation of FCV was observed upon reaction for two hours. In this case, overall, the most superior effect was obtained.

TABLE 5

| Overall results of antimicrobial activity of mixture of ginseng extract and green tea extract weight ratio of 66:34 | | | |
|---|---|---|---|
| Test strain samples | *E. coli* (24 hr) | *Yellow Staphylococcus Aureus* (3 hr) | FCV |
| (Example 3) 66:34 | 99.99% | 100% | 100% (2 hr) |

**<Example 6: Preparation of antimicrobial filter and evaluation of antimicrobial performance>**

(1) Preparation of antimicrobial filter

An air conditioning filter was produced using a zebra-shaped filter (produced by Lyusen Corporation) as a carrier. An antimicrobial filter was produced by mixing the produced ginseng extract and the green tea extract with the antimicrobial substance, the binder and the like shown in Table 6 at respective concentrations. A great amount of ethanol was added to the green tea extract because the green tea extract exhibits superior solution stability in an ethanol solvent. A natural extract may be added in an amount higher than 5% by weight, but was added in a reduced content as shown in the following table due to excessively deep color. As to the content of metal sol, the anti-fungi effect is advantageously improved when an overall copper concentration is increased. When 20% or more of a silicone-modified epoxy binder is used, stability of the solution is reduced. Accordingly, the silicone-modified epoxy binder is used in conjunction with a PVA #500 silicone binder.

TABLE 6

| Ingredients and ratios of antimicrobial filter | | |
|---|---|---|
| | Ingredients | Content (% by weight) |
| Natural extracts | Ginseng extract | 1.32 |
| | Green tea extract | 0.68 |
| Antimicrobial substance | Glutaraldehyde | 1 |
| Binder | Silicone-modified epoxy binder | 15 |
| | polyvinyl alcohol(PVA #500) silicone binder | 10 |
| Metal sol | Ca, Zn, Cu, Ag sol | 10 |
| Solvent | Pure water | 12 |
| | Ethanol | 50 |
| Total | | 100 |

(2) Evaluation of filter performance

The antimicrobial effect was measured using the produced antimicrobial filter and a filter was produced only using 2% by weight of the ginseng extract without using the green tea extract among the ingredients of Comparative Example and testing by comparison was performed. Results measured after 10 minutes of the antimicrobial activity are shown in the following Table 7.

TABLE 7

| Tests strains Configuration | E. coli | Yellow Staphylococcus Aureus | FCV | Antifungal activity |
|---|---|---|---|---|
| Ginseng extract alone | 99.91% | 99.93% | 99.20% | Grade 0 |
| Mixture (66:34) of ginseng extract and green tea extract | 100% | 100% | 100% | Grade 0 |

As can be seen from Table 7 above, the antimicrobial filter according to the present invention eliminated 100% of E. coli and yellow Staphylococcus Aureus upon reaction for 15 minutes and inactivated 100% of FCV, and had an antifungal activity of grade zero, thus exhibiting superior effects as compared to the filter using the ginseng extract alone.

As apparent from the fore-going, the present invention provides an antimicrobial composition containing a ginseng extract and a green tea extract, an antimicrobial filter and a method for manufacturing the same which maximize maximum antimicrobial and antiviral effects and exhibit antimicrobial and antiviral effects using a single filter, thus advantageously having little pressure loss and enabling production at low cost as compared to the related art using two filers.

It will be apparent to those skilled in the art that various modifications and variations can be made in the present invention without departing from the scope of the inventions. Thus, it is intended that the present invention covers the modifications and variations of this invention provided they come within the scope of the appended claims and their equivalents.

**Claims**

1.  An antimicrobial composition for an air conditioning filter, wherein the composition comprises a ginseng extract and a green tea extract, the ginseng extract and the green tea extract being present in a weight ratio of 60:40 to 70:30.

2.  The antimicrobial composition according to claim 1, wherein the ginseng extract is selected from the group consisting of a ginseng leaf extract, a ginseng rootlet extract, a ginseng main root extract and a combination thereof.

3.  The antimicrobial composition according to claim 1 or 2, wherein the ginseng extract is selected from the group consisting of a ginseng extract, a concentrated ginseng extract, a dried ginseng extract and a combination thereof and the green tea extract is selected from the group consisting of a green tea extract, a concentrated green tea extract, a dried green tea extract and a combination thereof.

4.  A method for preparing the antimicrobial composition for an air conditioning filter according to any of preceding claims, the method comprising:

    preparing a ginseng extract;
    preparing a green tea extract; and
    mixing the ginseng extract with the green tea extract,
    wherein the mixing is carried out by mixing the ginseng extract with the green tea extract at a weight ratio of 60:40 to 70:30.

5.  The method according to claim 4, wherein each of the preparation of the ginseng extract and the preparation of the green tea extract comprises powderizing the prepared extract and the powderizing is carried out by concentrating each of the extracts under reduced pressure.

6.  The method according to claim 4 or 5, wherein the preparation of the ginseng extract comprises:

    grinding any one selected from the group consisting of ginseng leaves, ginseng rootlets, ginseng main roots and a combination thereof to prepare ground ginseng;
    mixing the ground ginseng with distilled water and sterilizing the resulting mixture; and
    drying the mixture and extracting the dried mixture with a solvent.

7. The method according to any of claims 4 to 6, wherein the preparation of the green tea extract comprises:

grinding green tea to prepare ground green tea;
mixing the ground green tea with distilled water and sterilizing the resulting mixture; and
drying the mixture and extracting the dried mixture with a solvent.

8. An antimicrobial filter comprising the antimicrobial composition for an air conditioning filter either according to any of claims 1 to 3 or prepared by the method of any of claims 4 to 7.

9. The antimicrobial filter according to claim 8, wherein the antimicrobial filter further comprises any one selected from the group consisting of an antimicrobial substance, a binder, a metal and a combination thereof.

10. The antimicrobial filter according to claim 9, wherein the antimicrobial substance comprises any one selected from the group consisting of glutaraldehyde, phthalaldehyde (PA), potassium disulfite (PD), bronopol, sodium deoxycholate, quaternary ammonium and a combination thereof.

11. The antimicrobial filter according to claim 9 or 10, wherein the binder comprises any one selected from the group consisting of polyvinylpyrrolidone iodine (PVPI), norspermidine (NS, bis(3-aminopropyl)octylamine) and a combination thereof.

12. The antimicrobial filter according to claim 9 to 10, wherein the binder comprises any one selected from the group consisting of a silicone-modified epoxy resin, a silicone-modified acrylic resin, a silicone-modified urethane resin, a silicone-modified vinyl resin, a polyvinyl alcohol silicone resin, a urethane resin, an acrylic resin, a vinyl resin, a silicone resin and a combination thereof.

13. The antimicrobial filter according to any of claims 9 to 12, wherein the metal comprises any one selected from the group consisting of Ag, Cu, Zn, Ca, Mn and a combination thereof.

14. The antimicrobial filter according to any of claims 8 to 13, wherein the antimicrobial filter is suitable for being used for an air conditioner, an air cleaner, a refrigerator or a facial treatment device.

Europäisches
Patentamt

European
Patent Office

Office européen
des brevets

# EUROPEAN SEARCH REPORT

Application Number

EP 14 18 6253

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2011 0076347 A (REPUBLIC KOREA MAN RURAL DEV [KR]) 6 July 2011 (2011-07-06) * paragraphs [0035], [0036], [0039], [0040], [0049], [0050]; claims 1-10 * | 1-7 | INV. A01N65/08 A61K36/82 B01D46/00 A61K36/258 A01N25/34 A01P1/00 |
| X | KR 2005 0043105 A (CHANG YUN MI [KR]; GUM DONG IL [KR]; KIM YONG HI [KR]; KOREA GINSENG F) 11 May 2005 (2005-05-11) * claims 1-7 * | 1-4 | |
| X | KR 2013 0098051 A (IAC IN NAT UNIV CHUNGNAM [KR]) 4 September 2013 (2013-09-04) * paragraphs [0015] - [0019], [0032] - [0035]; claims 1-9 * | 1-3,8,14 | |
| X | KR 2004 0060271 A (JUNG KYOO JIN) 6 July 2004 (2004-07-06) * abstract * | 1-4 | |
| Y | * paragraphs [0035] - [0036], [0042]; table 1 * | 8-14 | |
| | | | TECHNICAL FIELDS SEARCHED (IPC) |
| Y | US 2013/199234 A1 (KIM EUNJEONG [KR] ET AL) 8 August 2013 (2013-08-08) * paragraphs [0123] - [0128]; claims 1-10 * | 8-14 | A01N A61K B01D |
| Y | KR 2009 0098570 A (KIM CHANG HEE [KR]) 17 September 2009 (2009-09-17) * abstract * * claim 1 * | 8-14 | |

-/--

The present search report has been drawn up for all claims

| Place of search | Date of completion of the search | Examiner |
|---|---|---|
| Munich | 9 January 2015 | Sawicki, Marcin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

**EUROPEAN SEARCH REPORT**

Application Number

EP 14 18 6253

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Jeff Nadeau: "Effects of the Combinations of Ginseng with Green Tea and Coriander on the Prohibition of E. coli Growth", Macomb Mathematics Science Technology Center , 30 May 2008 (2008-05-30), XP002734252, Retrieved from the Internet: URL:http://www.wcs.k12.mi.us/mmstc/student s/research/biology/2012/potis%20-%20wlodek .asp [retrieved on 2015-01-08] * sentences 15-18 * ----- | 8-14 | |

TECHNICAL FIELDS
SEARCHED        (IPC)

The present search report has been drawn up for all claims

| Place of search | Date of completion of the search | Examiner |
|---|---|---|
| Munich | 9 January 2015 | Sawicki, Marcin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.............................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 14 18 6253

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2015

| Patent document cited in search report | | Publication date | Patent family member(s) | | Publication date |
|---|---|---|---|---|---|
| KR 20110076347 | A | 06-07-2011 | NONE | | |
| KR 20050043105 | A | 11-05-2005 | NONE | | |
| KR 20130098051 | A | 04-09-2013 | NONE | | |
| KR 20040060271 | A | 06-07-2004 | NONE | | |
| US 2013199234 | A1 | 08-08-2013 | CN | 103239948 A | 14-08-2013 |
| | | | EP | 2623128 A1 | 07-08-2013 |
| | | | KR | 20130090688 A | 14-08-2013 |
| | | | US | 2013199234 A1 | 08-08-2013 |
| KR 20090098570 | A | 17-09-2009 | NONE | | |

EPO FORM P0459

For more details about this annex : see Official Journal of the European Patent Office, No. 12/82